(19) **Europäisches Patentamt / European Patent Office / Office européen des brevets**

(11) **EP 3 364 371 A1**

(12) **EUROPEAN PATENT APPLICATION**

(43) Date of publication:
**22.08.2018  Bulletin 2018/34**

(21) Application number: **17181650.7**

(22) Date of filing: **17.07.2017**

(51) Int Cl.:
**G06T 7/00** (2017.01)        **A61B 3/00** (2006.01)
**G06K 9/00** (2006.01)        **G06T 7/64** (2017.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.02.2017  KR 20170021789**

(71) Applicant: **PIXEL Display Inc.**
**Seoul 05719 (KR)**

(72) Inventors:
• **KWON, Tae Hyeon**
  **21124 Incheon (KR)**
• **LEE, Hyun Ho**
  **11949 Gyeonggi-do (KR)**

(74) Representative: **Zardi, Marco**
**M. Zardi & Co. SA**
**Via Pioda 6**
**6900 Lugano (CH)**

Remarks:
Amended claims in accordance with Rule 137(2) EPC.

(54) **USER DEVICE, SERVER, AND COMPUTER PROGRAM STORED IN COMPUTER-READABLE MEDIUM FOR DETERMINING VISION INFORMATION**

(57)    Disclosed herein are a user device, server, and computer program for determining vision information. The computer program is stored in a computer-readable storage medium including encoded instructions. When executed by one or more processors of a computer system, the computer program causes the one or more processors to perform operations, the operations comprising the operations of: allowing the operation of a lighting unit to be controlled in response to a photographing request; allowing a photographing unit to acquire an image of a pupil of a user in response to the photographing request; allowing intensity profile information regarding the diameter of the pupil to be acquired from the image of the pupil; allowing crescent length information to be determined from the intensity profile information; and allowing the vision information of the user to be determined at least partially based on the crescent length information.

```
                      START
                        │
                        ▼
   ┌──────────────────────────────────────────┐
   │   ALLOW A LIGHT MODULE TO BE CONTROLLED   │── S110
   └──────────────────────────────────────────┘
                        │
                        ▼
   ┌──────────────────────────────────────────┐
   │   ALLOW A PHOTOGRAPHED IMAGE INCLUDING AN │── S120
   │   IMAGE OF A PUPIL OF A USER TO BE ACQUIRED│
   └──────────────────────────────────────────┘
                        │
                        ▼
   ┌──────────────────────────────────────────┐
   │       ALLOW INTENSITY PROFILE INFORMATION │── S130
   │   REGARDING THE DIAMETER OF THE PUPIL TO BE│
   │        ACQUIRED FROM THE IMAGE OF THE PUPIL│
   └──────────────────────────────────────────┘
                        │
                        ▼
   ┌──────────────────────────────────────────┐
   │  ALLOW CRESCENT LENGTH INFORMATION TO BE  │── S140
   │ DETERMINED FROM THE INTENSIVE PROFILE INFORMATION│
   └──────────────────────────────────────────┘
                        │
                        ▼
   ┌──────────────────────────────────────────┐
   │         ALLOW THE VISION INFORMATION      │── S150
   │       OF THE USER TO BE DETERMINED        │
   └──────────────────────────────────────────┘
                        │
                        ▼
                      END
```

FIG.4

EP 3 364 371 A1

**Description**

BACKGROUND

1. Technical Field

The present invention relates generally to the field of image analysis, and more particularly to the determination of the vision information of a user from an image of an eyeball.

2. Description of the Related Art

Recently, the popularization of user devices, such as smartphones, tablet personal computers (PCs), etc., the construction of information technology (IT) infrastructures have proliferated rapidly. In general, the display units of user devices display various types of text and images, and thus users are exposed to the displays of user devices regardless of time and location. Accordingly, the vision of people today has been gradually declining.

Generally, the vision of a person is measured in a hospital, a health center, an optician's shop, or the like in such a manner that an examinee views pluralities of characters and shapes on an eye chart while maintaining a predetermined distance and makes responses in compliance with an examiner's instructions. This conventional vision measurement method is disadvantageous in that the reliability of vision measurement values is low. Furthermore, the conventional vision measurement method is inconvenient in that people must visit places where vision can be measured, such as hospitals, or the like.

Therefore, there is a huge demand for convenient and accurate vision measurement.

SUMMARY

The present invention has been conceived to overcome the above-described disadvantages, and an object of the present disclosure is to enable the vision information of a user to be determined from an image of an eyeball of the user.

According to an aspect of the present disclosure, there is disclosed a computer program stored in a computer-readable storage medium including encoded instructions. When executed by one or more processors of a computer system, the computer program causes the one or more processors to perform operations, the operations comprising the operations of: allowing the operation of a lighting unit to be controlled in response to a photographing request; allowing a photographing unit to acquire an image of a pupil of a user in response to the photographing request; allowing intensity profile information regarding the diameter of the pupil to be acquired from the image of the pupil; allowing crescent length information to be determined from the intensity profile information; and allowing the vision information of the user to be determined at least partially based on the crescent length information.

The operation of allowing a photographing unit to acquire an image of a pupil of a user may include the operations of: allowing the image of the pupil of the user to be photographed in response to a program execution request; and allowing the image of the pupil of the user, photographed by the photographing unit in response to the photographing request, to be stored in response to the program execution request.

The computer program may further include the operation of allowing the location of an eyeball including the pupil to be determined from the photographed image.

The operation of allowing crescent length information to be determined may include the operations of: analyzing a variation in intensity value intensity values from the intensity profile information; analyzing the direction of the variation; and determining at least one of vertical and horizontal lengths of a crescent at least partially based on the variation.

The computer program may further include the operation of allowing the image of the pupil to be displayed on a 3D graph.

The vision information of the user may be determined additionally based on at least one of the radius length of the pupil, the distance from the photographing unit to the pupil, and the distance from the photographing unit to the lighting unit.

The vision information of the user may be determined additionally based on at least one of a PSF algorithm, an optical transfer function algorithm, an LSF algorithm, and a Strehl ratio algorithm.

The computer program may further include the operation of determining the optimization information of the display unit of a user-related user device at least partially based on the vision information of the user.

The computer program may further include the operations of: allowing the photographing unit to acquire a second image of the pupil of the user in response to a second photographing request; comparing the image of the pupil acquired in response to the photographing request with the second image of the pupil acquired in response to the second photographing request; and determining whether the pupil has astigmatism at least partially based on the comparison; and the image of the pupil and the second image of the pupil may be photographed and acquired in different directions.

According to another aspect of the present disclosure, there is disclosed a user device. The user device includes: at least one lighting unit; at least one photographing unit; a control unit configured to be allowed to control the operation of the lighting unit in response to a photographing request; a photographed image acquisition unit configured to be allowed to allow the photographing unit to acquire an image of a pupil of a user in response to the photographing request; an intensity profile information acquisition unit configured to be allowed to acquire intensity profile information regarding the diameter of the pupil from the image of the pupil; a crescent length information determination unit configured to be allowed to determine crescent length information from the intensity profile information; and a vision information determination unit configured to be allowed to determine the vision information of the user at least partially based on the crescent length information.

According to another aspect of the present disclosure, there is disclosed a server. The server includes: an intensity profile information acquisition unit configured to be allowed to acquire intensity profile information regarding the diameter of a pupil from an image of the pupil of a user acquired in response to a photographing request; a crescent length information determination unit configured to be allowed to determine crescent length information from the intensity profile information; and a vision information determination unit configured to be allowed to determine the vision information of the user at least partially based on the crescent length information.

The image of the pupil of the user may be acquired in response to operation of a lighting unit controlled in response to the photographing request.

BRIEF DESCRIPTION OF THE DRAWINGS

The above and other objects, features and advantages of the present disclosure will be more clearly understood from the following detailed description taken in conjunction with the accompanying drawings, in which:

FIG. 1 is a diagram showing a system in which various embodiments of the present disclosure may be implemented;

FIG. 2 is a block diagram of a user device according to embodiments of the present disclosure;

FIG. 3 is a block diagram of a server according to embodiments of the present disclosure;

FIG. 4 is a flowchart showing a vision information determination method according to embodiments of the present disclosure;

FIG. 5 is a view showing examples of pupil image information according to embodiments of the present disclosure;

FIG. 6 is a view showing examples of intensity information regarding pupils according to the embodiments of the present disclosure;

FIG. 7 is a view showing examples of intensity information regarding pupils according to the embodiments of the present disclosure;

FIG. 8 is a view showing examples of a first image of a pupil of a user and a second image of the pupil of the user according to embodiments of the present disclosure;

FIG. 9 is a flowchart showing a method of controlling display according to the embodiments of the present disclosure;

FIGS. 10 and 11 are views showing display optimized for a user according to the embodiments of the present disclosure;

FIG. 12 is a view showing the way that one or more user devices are optimized based on the eye examination information of a user according to embodiments of the present disclosure; and

FIGS. 13 to 16 are views showing user interfaces that may be displayed on a user device according to embodiments of the present disclosure.

DETAILED DESCRIPTION

Various embodiments will be described with reference to the accompanying drawings below. In the present specification, various descriptions are presented to provide the understanding of the present disclosure. However, it will be apparent that these embodiments can be practiced without requiring the specific descriptions. In the embodiments, well-known components are provided in the form of block diagrams in order to facilitate descriptions of the embodiments.

The terms "component," "module," and "system" used herein refer to a computer-related entity, hardware, firmware, software, the combination of hardware and software, or software in execution. For example, the term "component" may be, but is not limited to, a process running on a processor, a processor, an object, an execution thread, a program, and/or a computer. By way of example, both an application running on a computing device and the computing device may be a component. One or more components may reside within a process and/or an execution thread, and a component may be localized on one computer and/or distributed across two or more computers. In addition, these components may be executed from various computer readable media having various data structures stored thereon. The components may communicate by way of local and/or remote processes, for example, in accordance with signals having one or more data packets (e.g., data from one component interacting with another component in a local system, a distributed system, and/or across a network, such as the Internet).

The term "or" used herein is intended to refer to an inclusive "or" rather than an exclusive "or". That is, unless specified otherwise or clear from the context, "X uses A or B" is intended to refer to any of natural inclusive permutations. That is, if X uses A, X uses B, or X uses both A and B, "X uses A or B" is satisfied in any one of the foregoing examples. Furthermore, it should be understood that the term "and/or" used herein refers to and includes any and all combinations of one or more of associated listed items.

It should be understood that the terms "include" and/or "including" used herein specify the presence of stated features, integers, steps, operations, elements, and/or components, but do not preclude the presence or addition of one or more other features, integers, steps, operations, elements, components, and/or groups thereof. The singular forms "a," "an," and "the" are intended to include the plural forms as well, unless the context clearly indicates otherwise.

The terms "information" and "data" used herein may be often used to be interchangeable with each other.

The description herein is presented to enable those skilled in the art to use and practice the invention. Various modifications to the disclosed embodiments will be readily apparent to those skilled in the art and the general principles defined herein may be applied to other embodiments and applications without departing from the spirit and scope of the present invention. Accordingly, the present invention is not intended to be limited to the disclosed embodiments, but should be interpreted in the broadest sense consistent with the principles and features disclosed herein.

Embodiments of the present disclosure will be described in detail with reference to the accompanying drawings below.

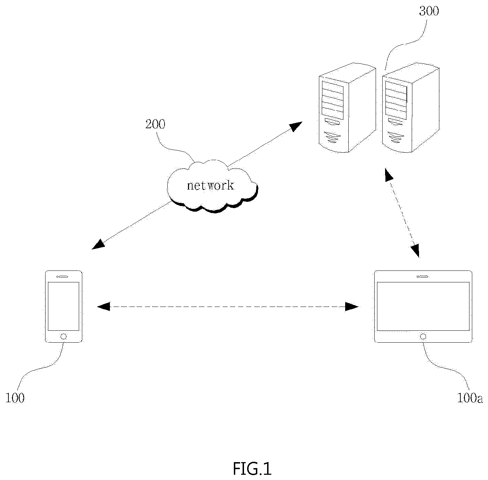

FIG. 1 is a diagram showing a system in which various embodiments of the present disclosure may be implemented.

The system according to embodiments of the present disclosure may include a user device 100, a network 200, and a server 300. The user device 100 and/or the server 300 according to the embodiments of the present disclosure may exchange data for the system according to the embodiments of the present disclosure over the network 200.

As shown in FIG. 1, the user device 100 and/or the server 300 may exchange data for the system according to the embodiments of the present disclosure with at least one user-related user device 100a over the network 200. The above-described at least one user-related device 100a may refer to another user device which is possessed by a user. The above-described at least one user-related device 100a may include a display unit. In the following description, the at least one user-related device 100a may be often interchanged with a second user device 100a of the user.

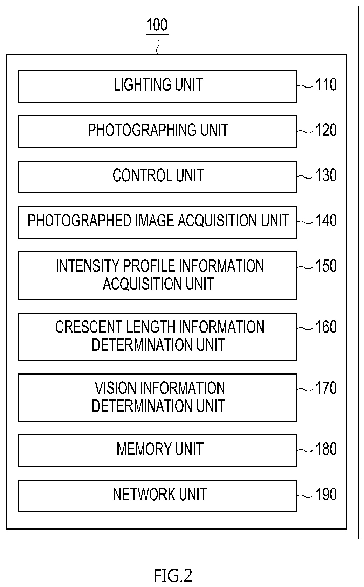

FIG. 2 is a block diagram of a user device 100 according to embodiments of the present disclosure.

Referring to FIG. 2, the user device 100 according to the embodiments of the present disclosure is now described. The user device 100 according to the embodiments of the present disclosure may include at least one lighting unit 110, at least one photographing unit 120, a control unit 130, a photographed image acquisition unit 140, an intensity profile information acquisition unit 150, a crescent length information determination unit 160, a vision information determination unit 170, a memory unit 180, and a network unit 190. Since the components shown in FIG. 2 are not essential, the user device 100 may be implemented to have a larger number of components or a smaller number of components.

The components will be described in sequence below.

The user device 100 according to the embodiments of the present disclosure may include the at least one lighting unit 110, and the at least one photographing unit 120.

In the embodiments of the present disclosure, the lighting unit 110 may emit light in response to a photographing request. The above-described lighting unit 110 may be, for example, a flash module installed inside the user device 100. The lighting unit 110 may be located within a predetermined distance from the photographing unit 120. The operations (the emission of light, flash duration, flash strength, etc.) of the lighting unit 110 may be controlled by the control unit 130.

In the embodiments of the present disclosure, the photographing unit 120 may be configured to acquire an image of a pupil of a user in response to a photographing request. The vision information of the user may be determined from the image of the pupil.

In greater detail, the photographing unit 120 may photographs the image of the pupil of the user in response to a program execution request. The image of the pupil of the user photographed by the photographing unit 120 may be stored in the memory unit 180 in response to the photographing request. For example, the user device 100 may include a computer application or software configured to be operated to an image photographed (and/or scanned) by the photographing unit 120.

The photographing unit 120 according to the embodiments of the present disclosure performs photographing in order to measure the vision of an eye, and may include a camera sensor configured to convert a photographed optical signal into an electrical signal and a signal processing unit configured to convert an analog image signal, photographed by the camera sensor, into digital data. However, the range of the rights of the present disclosure is not limited thereto.

According to the embodiments of the present disclosure, the user device 100 may include the control unit 130. This control unit 130 controls and processes all the operations of the user device 100.

For example, the control unit 130 may control the operations of the lighting unit 110 and/or the photographing unit 120. In greater detail, the control unit 130 may allow the photographing unit 120 to photograph the pupil of the user in response to program execution request. Furthermore, the control unit 130 may allow the lighting unit 110 to emit light and allow the photographed image acquisition unit 140 to acquire an image of a pupil of the user in response to a photographing request. In greater detail, the control unit 130 may allow the image of the pupil of the user, photographed by the photographing unit 120 in response to a program execution request, to be stored in the memory unit 180 in response to a photographing request. The above-described program execution request may include any requests, such as a program initiation request, a photographing preparation request, etc., received before the photographing request.

In the embodiments of the present disclosure, the control unit 130 may determine the location of an eyeball, including the pupil, from the image photographed by the photographing unit 120. For example, cornea reflected light candidates for the acquired image are extracted, and the location of the pupil may be determined based on the extracted cornea reflected light candidates. Alternatively, the acquired image may be divided into a plurality of blocks, and the location of the pupil may be determined by comparing pixels within the resulting blocks. The above-described method of determining the location of an eyeball including a pupil is merely an example according to the embodiments of the present disclosure, but the range of rights of the present disclosure is not limited thereto. In the embodiments of the present disclosure, the photographing unit 120 may acquire the image of the pupil of the user with high quality by being focused on the location of the eyeball.

The control unit 130 according to the embodiments of the present disclosure may include, for example, not only hardware, such as a center processing unit, a web server or the like, but also software, such as an operating system program, a control program, etc.

The control unit 130 can communicate with all the above-described components and components to be described later, and thus can systematically control the operations of the components.

As described above, the photographed image acquisition unit 140 allows the photographing unit 120 to acquire the image of the pupil of the user in response to the photographing request.

In the embodiments of the present disclosure, the image of the pupil of the user may be photographed by the photographing unit 120 in response to a program execution request, and may be stored in response to a photographing request. In other words, in the embodiments of the present disclosure, the photographed image acquisition unit 140 may acquire the image of the pupil the moment the lighting unit 110 emits light. This can prevent the pupil of the user from being contracted or prevent a red-eye effect from occurring, thereby enabling the vision information of the user to be determined from the image of the pupil.

In the embodiments of the present disclosure, the intensity profile information acquisition unit 150 may be allowed to acquire intensity profile information regarding the diameter of the pupil from the image of the pupil.

In the embodiments of the present disclosure, the intensity profile information refers to the intensity values of the diameter of the pupil.

In the embodiments of the present disclosure, the intensity profile information may be acquired by presenting the image of the pupil of the user on a 3D graph. In greater detail, the image of the pupil may be represented on an X axis, a Y axis, and a Z axis. In the embodiments of the present disclosure, the intensity profile information may be acquired from the image of the pupil represented on an X axis, a Y axis, and a Z axis.

In the embodiments of the present disclosure, the crescent length information determination unit 160 may be allowed to determine crescent length information from the intensity profile information.

In the embodiments of the present disclosure, the crescent length information may be the vertical length of a crescent which is determined at least partially based on a variation in intensity value intensity values after the variation has been analyzed from the intensity profile information acquired by the intensity profile information acquisition unit 150. The above-described variation may be analyzed in connection with the direction thereof. As another example, the crescent length information may be the horizontal length of a crescent which is determined at least partially based on a variation in intensity values. For example, the crescent length information may be acquired by applying a differential equation to the intensity profile information, but the range of rights of the present disclosure is not limited thereto.

In the embodiments of the present disclosure, the vision information determination unit 170 may be allowed to determine the vision information of the user at least partially based on crescent length information. The vision information of the user may be determined additionally based on at least one of the length of the radius of the pupil, the distance from the photographing unit to the pupil, and the distance from the photographing unit to the lighting unit. Furthermore, the vision information of the user may be determined additionally based on at least one of a PSF algorithm, an optical transfer function algorithm, an LSF algorithm, and a Strehl ratio algorithm. It will be apparent to those skilled in the art that to determine the vision information of the user at least partially based on the crescent length information, another unmentioned technology may be adopted.

As described above, the vision information of the user may be determined from the image of the pupil of the user acquired by the user device 100. The optimization information of the display unit of the user-related user device may be determined at least partially based on the vision information of the user.

According to the embodiments of the present disclosure, the image of the pupil of the user may be acquired for the authentication of the user. In other words, according to the embodiments of the present disclosure, even when the image of the pupil is not intentionally acquired, the vision information may be determined from the image of the pupil acquired for authentication, thereby increasing the convenience of the user.

The memory 180 according to the embodiments of the present disclosure stores various types of information which are generated during the processing performance of the system according to the embodiments of the present disclosure. For example, the memory 180 may store the acquired image of the pupil information. Furthermore, the memory unit 180 may store the display optimization information for the user, information about the at least one user-related user device including a display unit, etc. In particular, the memory unit 180 may store various data and programs for the provision of display of an appropriate font, appropriate resolution, and/or the like to the user at least partially based on the eye examination information of the user including determined vision information. As another example, eye examination information and distance-based display optimization matching information may be stored in the memory unit 180.

In other words, the memory unit 180 may store a program for the operation of the above-described control unit 130, and may temporarily or permanently store input/output data.

The memory unit 180 may include at least one type of storage medium selected from among flash memory, a hard disk, a multimedia card, card-type memory (for example, SD or XD memory, or the like), random access memory (RAM), static random access memory (SRAM), read-only memory (ROM), electrically erasable programmable read-only memory (EEPROM), programmable read-only memory (PROM), magnetic memory, a magnetic disk, and an optical disk.

Referring to FIG. 2, although the memory unit 180 is provided inside the user device 100 in the present embodiment, the user device 100 may operate in conjunction with web storage configured to perform the storage function of the memory unit 180 over the Internet.

Various types of information used for the above-described operations and operations to be described later may be exchanged with the server 200 and/or the at least one user-related user device 100a via the network unit 190.

The network unit 190 may include a wired/wireless Internet module for network connection as a network module. A wireless Internet technology for the wireless Internet module may include Wireless LAN (WLAN), Wi-Fi, Wireless broadband (Wibro), World Interoperability for Microwave Access (Wimax), High Speed Downlink Packet Access (HSDPA), etc. A wired Internet technology for the wired Internet module may include Digital Subscriber Line (XDSL), Fibers to the home (FTTH), Power Line Communication (PLC), etc. However, it will be apparent to those skilled in the art that the range of rights of the present disclosure is not limited thereto.

The network unit 190 according to the embodiments of the present disclosure may additionally include a short range communication module, and may exchange data with another user device which is located within a relatively short distance from the user device 100 and includes a short range communication module. A short range communication technology for the short range communication module may include Bluetooth, Radio Frequency Identification (RFID), Infrared Data Association (IrDA), Ultra Wideband (UWB), ZigBee, etc., but the range of rights of the present disclosure is not limited thereto.

According to the embodiments of the present disclosure, although not shown in the drawing, the user device 100 may further include a data processing unit. The data processing unit may include a transmitter configured to encode and modulate a transmitted signal, a receiver configured to demodulate and decode a received signal.

According to the embodiments of the present disclosure, although not shown in the drawing, the user device 100 may further include at least one of an audio processing unit, an RF unit, and a display unit.

The audio processing unit according to the embodiments of the present disclosure functions to play back a received audio signal input and output to and from the control unit 130, or functions to transmit a transmitted/received audio signal generated by a microphone.

The RF unit according to the embodiments of the present disclosure may perform a wireless communication function. The RF unit may include an RF transmitter configured to up-convert the frequency of a transmitted signal and amplify the transmitted signal, and an RF receiver configured to low-noise amplify a received signal and down-convert the frequency of the received signal.

The display unit according to the embodiments of the present disclosure may display the image of the pupil of the user photographed by the photographing unit 120 and/or user interfaces.

The display unit may include at least one of a liquid crystal display (LCD), a thin film transistor-liquid crystal display (TFT LCD), an organic light-emitting diode (OLED), a flexible display, and a 3D display. Of these displays, some display modules may be transparent-type display modules or light transmission-type display modules which allow the outside to be viewed therethrough. These are referred to as transparent display modules. A representative example of such a transparent display module is a transparent OLED (TOLED) or the like.

In an embodiment of the present disclosure, two or more display units may be present according to the implementation of the user device 100. For example, in the user device 100, a plurality of displays may be disposed on a single surface in a separate or integrated form, or may be disposed on different surfaces. For example, the display unit may include both or any one of a display user disposed on the upper end portion of the device 100 and a display disposed on the lower end portion of the device 100. However, the locations at which the above-described display unit is disposed are merely examples, and the display unit may be disposed at various locations according to a need for design or a visual effect.

In an embodiment of the present disclosure, the display unit may be composed of a touch screen implemented to receive the selection input of the user. The display unit composed of the touch screen may include a touch sensor. The touch sensor may be configured to convert a change, such as pressure applied to a specific portion of the display unit or capacitance generated in a specific portion of the display unit, into an electrical input signal. The touch sensor may be configured to detect not only a touch location and a touch area but also a touch pressure. When touch input is applied to the touch sensor, a signal(s) corresponding to the touch input is transferred to a touch controller. The touch controller processes the signal(s), and transmits corresponding data to the control unit 130. This enables the control unit 130 to recognize the portion of the display unit which has been touched.

According to the above-described embodiments of the present disclosure, display settings for screens to be displayed on the display unit may be determined to be adjusted at least partially based on display optimization information for the user determined at least partially based on user information including the eye examination information of the user.

According to the embodiments of the present disclosure, the user device 100 may include at least one of a proximate sensor, an infrared sensor, an RF sensor, a gyro sensor, and an ultrasonic sensor in order to measure distance, but the range of rights of the present disclosure is not limited thereto.

According to the embodiments of the present disclosure, the user device 100 may include an input unit (for example, a key input unit). The input unit includes keys configured to receive number and text information. According to the embodiments of the present disclosure, the input unit may include keys configured to execute an application configured to determine vision according to the embodiments of the present disclosure. Furthermore, the input unit may include a key configured to initiate the display settings adjusted according to the display optimization information.

The user device 100 according to the embodiments of the present disclosure may refer to any device capable of using a wireless connection mechanism, such as an electronic communication device, a TV, a navigation system, a camcorder, a camera, a user terminal, user equipment, a mobile device, a wireless communication-enabled PC, a mobile phone, a kiosk, a cellular phone, a cellular device, a cellular terminal, a subscriber unit, a subscriber station, a mobile station, a terminal, a remote station, a PDA, a remote terminal, an access terminal, a user agent, a portable device having a wireless access function, or a wireless modem, but is not limited thereto. Furthermore, the user device 100 may refer to any device capable of using a wired connection mechanism, such as a wired facsimile, a PC having a wired modem, a wired phone, or a terminal capable of wired communication, but is not limited thereto. For example, the user device 100 according to the embodiments of the present disclosure may be a remote control configured to implement the system according to the embodiments of the present disclosure.

According to the embodiments of the present disclosure, the user can measure vision via the photographing unit and lighting unit of the user device 100 (for example, a mobile phone, or the like), and thus can be conveniently provided with highly accurate vision information via the user device carried by the user.

The user device 100 can exchange various types of information with the server 300 and/or the at least one user-related user device 100a by means of a web browser or mobile application.

Referring to FIG. 2, part and/or all of various embodiments described herein may be implemented in a computer or similar device-readable recording medium or storage medium by using, for example, software, hardware or the combination thereof.

The network 200 according to the embodiments of the present disclosure may use various wired communication systems, such as a Public Switched Telephone Network (PSTN) system, an x Digital Subscriber Line (xDSL) system, a Rate Adaptive DSL (RADSL) system, a Multi Rate DSL (MDSL) system, a Very High Speed DSL (VDSL) system, a Universal Asymmetric DSL (UADSL) system, a High Bit Rate DSL (HDSL) system, a Local Area Network (LAN) system, etc.

Furthermore, the network presented herein uses various wireless communication systems, such as a Code Division Multi Access (CDMA) system, a Time Division Multi Access (TDMA) system, a Frequency Division Multi Access (FDMA) system, an Orthogonal Frequency Division Multi Access (OFDMA) system, a Single Carrier-FDMA (SC-FDMA) system, and other systems.

The network according to one embodiment of the present disclosure may be composed of various types of networks, such as a wired network, a wireless network, etc., and may be composed of various communication networks, such as a Personal Area Network (PAN), a Wide Area Network (WAN), etc. Furthermore, the network may be the well-known World Wide Web (WWW), and may use a wireless transmission technology used for short range communication, such as Infrared Data Association (IrDA) or Bluetooth.

The technologies described herein may be used in not only the above-described networks but also other networks.

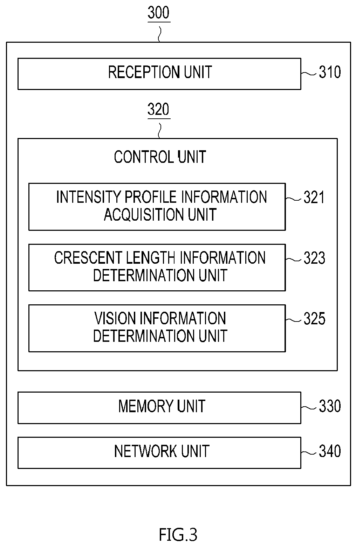

FIG. 3 is a block diagram of a server 300 according to embodiments of the present disclosure.

The server 300 according to the embodiments of the present disclosure may include a reception unit 310, a control unit 320, a memory unit 330, and a network unit 340. The control unit 320 according to the embodiments of the present disclosure may include an intensity profile information acquisition unit 321, a crescent length information determination unit 323, and a vision information determination unit 325. Although the above-described modules 321, 323 and 325 are shown as being included in the control unit 320 in FIG. 3, they may be present as separate modules.

In the embodiments of the present disclosure, the reception unit 310 may receive an image of a pupil of a user from the user device 100. Furthermore, the server 300 may receive the image of the pupil and the eye examination information of the user from another server (not shown; for example, a hospital server, an optician's shop server, or the like).

In the embodiments of the present disclosure, the intensity profile information acquisition unit 321 may be allowed to acquire intensity profile information regarding the diameter of the pupil from the image of the pupil of the user acquired in response to a photographing request. In this case, the image of the pupil of the user may be acquired in response to the operation of the lighting unit controlled in response to the photographing request.

In the embodiments of the present disclosure, the crescent length information determination unit 323 may be allowed to determine the crescent length information from the intensity profile information.

In the embodiments of the present disclosure, the vision information determination unit 325 may be allowed to determine the vision information of the user at least partially based on the crescent length information.

Since the details of the server 300 according to the embodiments of the present disclosure correspond to the method performed by the user device 100, detailed descriptions thereof are omitted below.

The components included in the system shown in FIG. 1 are merely examples, and thus some of the components may constitute the system or one or more components additional to the components may be included in the system.

For example, the system according to the embodiments of the present disclosure may include the above-described the at least one user-related user device 100a. The at least one user-related user device 100a may be connected to the user device 100 and/or server 300 over the network 200.

As described above, the term "at least one user-related user device" used herein may refer to anther user device possessed by the user. The at least one user-related user device 100a may include a display unit. In the following description, the at least one user-related user device 100a may be often interchanged with a second user device 100a of the user.

The user device 100a may refer to any device capable of using a wireless connection mechanism, such as an electronic communication device, a TV, a navigation system, a camera, a user terminal, user equipment, a mobile device, a wireless communication-enabled PC, a mobile phone, a kiosk, a cellular phone, a cellular device, a cellular terminal, a subscriber unit, a subscriber station, a mobile station, a terminal, a remote station, a PDA, a remote terminal, an access terminal, a user agent, a portable device having a wireless access function, or a wireless modem, but is not limited thereto.

Additionally, information transmitted and received by the system described with reference to FIG. 1 may be stored in the database or computer-readable storage medium of the user device 100, the server 300, and the at least one user-related user device 100a. The storage medium may include all types of storage media in which a program and data are stored such that a computer system can read them. According to one embodiment of the present disclosure, the medium may include read-only memory (ROM), random access memory (RAM), compact disk (CD)-ROM, digital video disk (DVD)-ROM, magnetic tape, a floppy disk, an optical data storage device, etc. Additionally, the medium may be distributed across systems connected by a network, and may store computer-readable codes and/or instructions in a distributed manner.

The various embodiments described herein may be implemented within a computer- or similar device-readable recording medium by using, for example, software, hardware, or the combination thereof.

According to hardware implementation, the embodiments described herein may be implemented using at least one of application specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, and other function performance electric units. In some cases, the embodiments described herein may be implemented as the control unit 190 itself.

According to software implementation, embodiments, such as processes and functions, described herein may be implemented as separate software modules. Each of the software modules may perform one or more functions and operations described herein. The software codes may be implemented as a software application written in an appropriate program language. The software codes may be stored in the memory 180, and may be executed by the control unit 190.

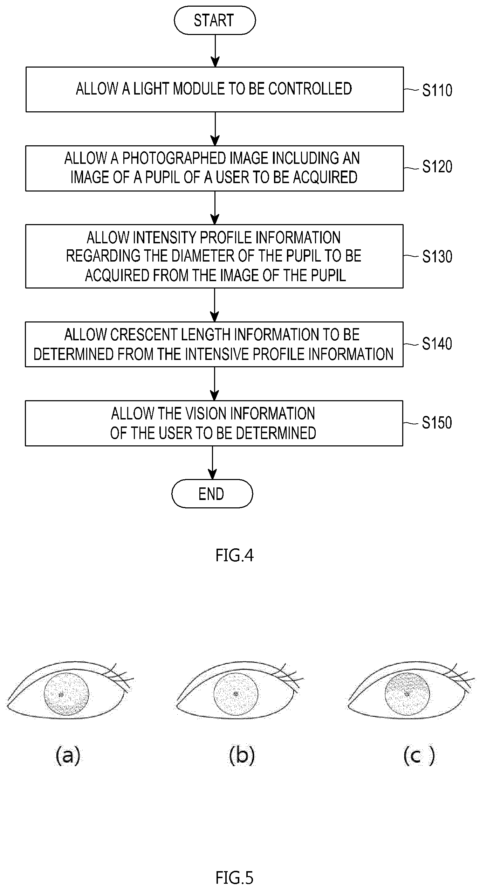

FIG. 4 is a flowchart showing a vision information determination method according to embodiments of the present disclosure.

Since the user device, the server and the computer program for the determination of vision information according to the embodiments of the present disclosure have been described with reference to FIGS. 1 to 3, the steps of the vision information determination method according to the embodiments of the present disclosure will be described with reference to FIG. 4 in brief below.

In the embodiments of the present disclosure, the operation of the lighting unit may be allowed to be controlled in response to a photographing request at step S110.

In the embodiments of the present disclosure, the photographing unit may be allowed to acquire an image of a pupil of a user in response to the photographing request at step S120.

FIG. 5 is a view showing examples of pupil image information according to embodiments of the present disclosure.

FIG. 5(a) shows an example of nearsightedness, FIG. 5(b) shows an example of normal vision, and FIG. 5(c) shows an example of farsightedness.

In the embodiments of the present disclosure, the image of the pupil of the user may be acquired the moment the lighting unit 110 emits light. For this purpose, the photographing unit 120 may photograph the user in response to program execution request, and may acquire a photographed image in response to the photographing request the moment the lighting unit 110 emits light.

In the embodiments of the present disclosure, intensity profile information regarding the diameter of the pupil may be allowed to acquire the image of the pupil from at step S130.

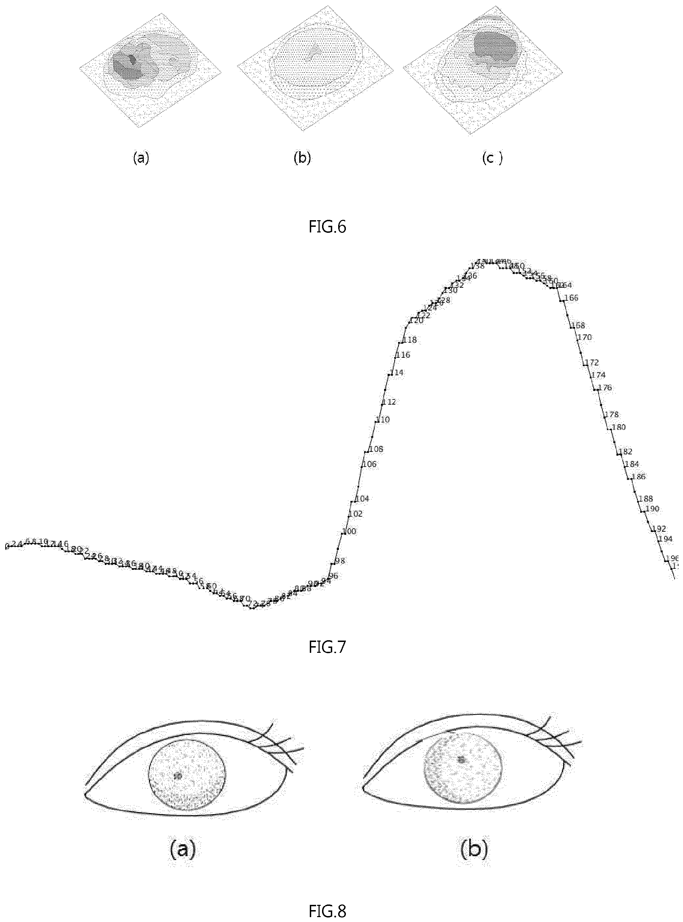

FIG. 6 is a view showing examples of intensity information regarding pupils according to the embodiments of the present disclosure.

FIG. 6 shows the intensities of images of the pupils on 3D graphs.

FIG. 6(a) shows the intensities of nearsightedness on a 3D graph, FIG. 6(b) shows the intensities of normal vision on a 3D graph, and FIG. 6(c) shown the intensities of farsightedness on a 3D graph. In other words, the images of the pupils acquired in the embodiments of the present disclosure may be represented along an X axis, a Y axis, and a Z axis, as shown in FIG. 6.

In the embodiments of the present disclosure, the crescent length information may be allowed to be determined from the intensity profile information at step S140.

FIG. 7 is a view showing examples of intensity information regarding pupils according to the embodiments of the present disclosure.

FIG. 7 indicates that crescent length information may be determined from the intensity profile information of the images of the pupils, such as those shown in FIGS. 6(a), 6(b) and 6(c). In this case, the crescent length information may refer to a crescent vertical length, and/or a crescent horizontal length.

In the embodiments of the present disclosure, the crescent length information may be determined at least partially based on a variation in intensity values analyzed from the intensity profile information. The direction of the variation in intensity values may be also analyzed. The variation in intensity values may vary on an upper side and a low side depending on an eyeball.

Referring to FIGS. 6 and 7, in the embodiments of the present disclosure, a variation in intensity values, such as that shown in FIG. 7, may be presented by applying a differential operation to intensity information shown on a 3D graph.

Referring to FIG. 7, the midpoint of the first decline curve from the right side of the graph may be estimated to be a crescent critical point.

In the embodiments of the present disclosure, the vision information of the user may be allowed to be determined at least partially based on the crescent length information at step S150.

In the embodiments of the present disclosure, the vision information of the user may be determined at least partially based on the crescent length information. The vision information of the user may be determined additionally based on at least one of the radius length of the pupil, the distance from the photographing unit to the pupil, and the distance from the photographing unit to the lighting unit. Furthermore, the vision information of the user may be determined additionally based on at least one of a PSF algorithm, an optical transfer function algorithm, an LSF algorithm, and a Strehl ratio algorithm. It will be apparent to those skilled in the art that to determine the vision information of the user at least partially based on the crescent length information, another unmentioned technology may be adopted.

In greater detail, the vision information of the user according to the embodiments of the present disclosure may be determined based on the difference between the radius of the pupil and a crescent length (for example, the vertical length of a determined crescent). The vision information of the user according to the embodiments of the present disclosure may be determined additionally based on the radius of the pupil, for example, ((2 x radius pupil) - the vertical length of the crescent)/(2 x the radius of the pupil).

As described above, the vision information of the user according to the embodiments of the present disclosure may be determined at least partially based on the following equation. In the following equation, R is a pupil radius length, A is the distance from the photographing unit to the pupil, D is dioptric defocus, E is the distance from the lighting unit to the photographing unit, and S is the vertical length of the crescent.

$$(2R - s)/2R = E(2ARD)$$

FIG. 8 is a view showing examples of a first image of a pupil of a user and a second image of the pupil of the user according to embodiments of the present disclosure.

In the case of astigmatism, light injected into an eye is not focused on a single focal point on a retina, but is scattered. Accordingly, in the case of astigmatism, a crescent may not be clear, but may appear in a blurred and wide form. Furthermore, in the case of astigmatism, when an image of a pupil is photographed at a different angle of the photographing unit, the direction of a crescent varies.

FIG. 8(a) is the first image of the pupil of the user. In greater detail, FIG. 8(a) is the image of the pupil of the user which is photographed when the user device 100 forms 0° with respect to a horizontal line.

FIG. 8(b) is the second image of the pupil of the user. In greater detail, FIG. 8(b) is the image of the pupil of the user which is photographed when the angle of the user device 100 is different (for example, when the angle of the user device 100 is disposed at the location rotated from the horizontal line by 90°).

Referring to FIGS. 8(a) and 8(b), in the case of the same user, when at least two images of the pupil are acquired with the user device 100 (for example, the photographing unit) disposed at a different angle, the direction of the crescent of the photographed image of the pupil may vary. In FIG. 8(a), the lower end of the pupil is clear, and, in FIG. 8(b), the left side of the pupil is clear. Alternatively, when at least two images of the pupil are acquired with the user device 100 disposed at a different angle as described above, the crescent of the acquired image of the pupil may not be clear, but may appear in a blurred and wide form. Alternatively, the direction of a crescent may appear in a different form. In this case, it may be determined that the pupil of the user has astigmatism.

In other words, according to the embodiments of the present disclosure, the operation of the lighting unit may be allowed to be controlled in response to a second photographing request. Furthermore, the photographing unit may acquire a second image of the pupil of the user in response to the second photographing request. Furthermore, the image of the pupil acquired in response to the photographing request may be compared with the second image of the pupil acquired in response to the second photographing request. In the embodiments of the present disclosure, the image of the pupil and the second image of the pupil may be photographed and acquired in different directions.

The above-described comparison between the two images of the pupil may be performed via the RGB analysis, image processing analysis and/or machine learning analysis of the horizontal length of the crescent, the vertical length of the crescent, a crescent distribution chart, etc. Whether the pupil has astigmatism may be determined at least partially based on the above-described comparison.

To determine whether the pupil has astigmatism from the acquired image of the pupil as described above, at least two images of the pupil photographed at different angles are required. Furthermore, the crescent distribution direction, location and size of an overall eyeball may be taken into consideration via RGB analysis, image processing, etc. in according to the above-described horizontal length and vertical length of the crescent.

In the embodiments of the present disclosure, the above-described method of determining the vision information of the user from the eyeball image may be learned via machine learning. In other words, the eye examination information from the acquired the image of the pupil may be determined using a network function learned from reference images of the pupil.

Recently, the popularization of user devices, such as smartphones, tablet PCs, etc., and the construction of IT infrastructures have proliferated rapidly. In general, the display units of user devices display various types of text and images, and thus users are exposed to the displays of user devices regardless of time and location. Accordingly, the vision of people today has gradually failing.

According to the above-described embodiments of the present disclosure, the user can conveniently measure his or her own vision without visiting a hospital, a health center, an optician's shop, or the like. Furthermore, the above-described embodiments enable more accurate vision to be measured than a conventional method of measuring vision based on an examinee's responses to pluralities of characters and shapes displayed on an eye chart used for the measurement of vision.

Some operation(s) of the described or shown operations may be omitted according to embodiments of the present disclosure. Furthermore, the operations shown in FIG. 4 are merely examples, and an additional operation may be included in the range of rights of the present disclosure.

Recently, with the development of IT technology, the popularization of user devices including display units (for example, notebook computers, smartphones, tablet PCs, etc.) has been increased. Accordingly, the number of user devices including display units possessed by each individual has been also increased.

According to statistics, the average period during which humans view display screens is about 400 minutes per day over the world. When cases where the eyes of users are unintentionally exposed to display screens are included, it is estimated that the average period during which humans view display screens is higher than the above period.

Accordingly, the number of people who wear glasses has also increased. Furthermore, the number of patients with dry eyes has increased two or more times over the past decade. In particular, over the past five years, the number of patients with glaucoma has recently increased two or more times among people in their teens or twenties who are frequently exposed to display screens, such as the display screens of PCs, smartphones, etc.

Optimized display may be provided to the user at least partially based on the vision information of the user determined according to the embodiments of the present disclosure.

The user device 100 according to the embodiments of the present disclosure may determine the optimization information of the display unit of the at least one user-related user device at least partially based on the vision information of the user. Furthermore, the user device 100 may be allowed to adjust the display settings of the user device at least partially based on the determined display optimization information. Furthermore, the user device 100 may determine to adjust the display settings of the at least one user-related user device at least partially based on the determined display optimization information.

In this case, the at least one user-related user device 100a may be another user device which is possessed by the user. As another example, the at least one user-related user device 100a may refer to another user device with which an account of the user is shared. In this case, the at least one user-related user device 100a may be configured to include at least one display unit.

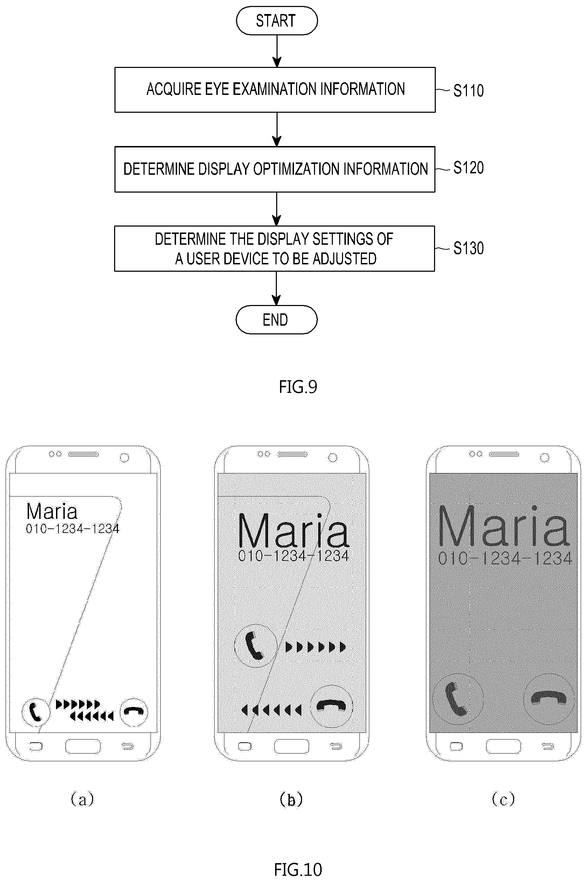

FIG. 9 is a flowchart showing a method of controlling display according to the embodiments of the present disclosure.

Some of the steps shown in FIG. 9 may be omitted according to an embodiment of the present disclosure. Furthermore, the steps shown in FIG. 9 are merely examples, and an additional step may be included in the range of rights of the present disclosure.

The steps shown in FIG. 9 may be performed by the user device 100. In greater detail, the following method may be performed by, for example, the individual modules of the user device 100.

As another example, the following steps may be performed by a computer-readable storage medium configured to control a display. Furthermore, the method shown in FIG. 9 may be performed by the hardware or OS of the server 300 (see FIG. 1) according to another embodiment of the present disclosure.

The above-described steps are merely examples according to an embodiment of the present disclosure. Accordingly, the above-described steps may include additional steps, or some steps may be omitted from the above-described steps. Furthermore, some steps may be added to and omitted from the steps shown in FIG. 9 as desired.

The method shown in FIG. 9 is described as being performed by the user device 100 below.

According to the embodiments of the present disclosure, eye examination information may be acquired at step S210.

The eye examination information according to the embodiments of the present disclosure may include vision information which is determined by the method of determining vision described with reference to FIGS. 1 to 8.

In greater detail, in the embodiments of the present disclosure, the eye examination information may refer to information which is obtained by an examination performed to check whether vision is good or bad, color blindness, the abnormality of an eye, or the like. The eye examination information according to the embodiments of the present disclosure may include at least one of a plurality of pieces of eyeball health information, including vision information, nearsightedness information, astigmatism information, weak eyesight information, color weakness information, and risk information, but the range of rights of the present disclosure is not limited thereto.

According to the embodiments of the present disclosure, the eye examination information may be received from, for example, the server 300.

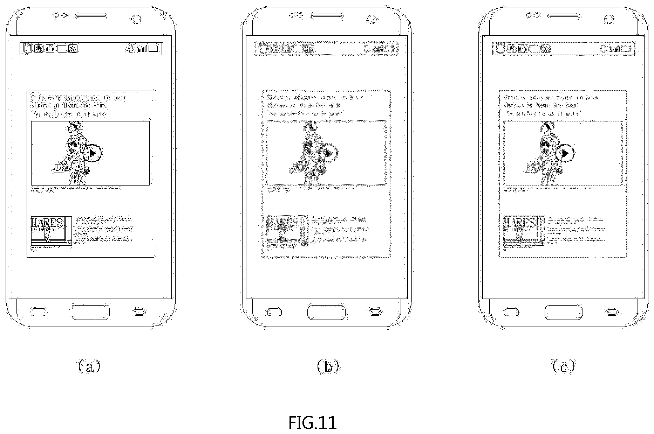

According to the embodiments of the present disclosure, the eye examination information may be generated at least partially based on the responses of a user to an eye examination interface including at least one of images and text used to perform eye examination on the user. In other words, a series of steps configured to acquire additional eye examination information in addition to the vision information determined by the method of determining vision according to the embodiments of the present disclosure may be performed by the user device 100. This is described with reference to FIGS. 11 to 12 below.

According to the embodiments of the present disclosure, display optimization information for the user may be determined at least partially based on user information including the eye examination information at step S220.

The display optimization information includes at least one of font type information, font size information, font brightness information, front contrast information, font-to-screen ratio information, screen brightness information, image size information, image brightness information, resolution information, and color correction information, but the range of rights of the present disclosure is not limited thereto.

The user information according to the embodiments of the present disclosure may include various types of information, such as user identification information, user age information, user age information, etc., in addition to the above-described eye examination information.

The display optimization information according to the embodiments of the present disclosure may be determined additionally based on at least one of user device attribute information, external environment information, and preference information.

In greater detail, the user device attribute information may include, for example, resolution information, photographing unit (i.e., a photographing module) information, lighting unit (i.e., a photographing light source) information, information about the distance from the photographing unit to the lighting unit, information about the type of user device, information about the size of the user device, etc., but the range of rights of the present disclosure is not limited thereto.

The external environment information according to the embodiments of the present disclosure may include, for example, information about the physical location of each of the user and the user device. Information about ambient light around the user or the user device may be included in the external environment information. Optionally or alternatively, the external environment information may include weather information regarding the physical location of the user, but the range of rights of the present disclosure is not limited thereto.

The preference information according to the embodiments of the present disclosure may be information corresponding to user feedback to the display optimization information for the user which is determined at least partially based on the user information including the eye examination information.

According to the embodiments of the present disclosure, the display settings of the user device may be determined to be adjusted at least partially based on the determined display optimization information at step S230.

According to the embodiments of the present disclosure, the display optimization information may be determined by considering the above-described information about various types of factors. Accordingly, optimized display can be provided to the user.

According to the embodiments of the present disclosure, to optimize display settings, the display settings may be automatically adjusted based on the display optimization information determined at least partially based on the user information including the eye examination information of the user without requiring that the user manually adjust the display settings. Accordingly, the convenience of the user can be improved.

Additionally, the display settings of the at least one user-related user device may be determined to be adjusted at least partially based on the determined display optimization information. Accordingly, a user using a plurality of user devices does not need to adjust the display settings of each of the user devices, and thus the convenience of the user can be improved.

The above description is merely an example according to an embodiment of the present disclosure, but unmentioned various examples may be included in the range of rights of the present disclosure.

FIGS. 10 and 11 are views showing display optimized for a user according to the embodiments of the present disclosure.

FIG. 10 shows views of display optimized for a user according to the embodiments of the present disclosure.

According to the embodiments of the present disclosure, display optimization information for the user may be determined at least partially based on user information including eye examination information. In this case, the eye examination information may include vision information which is determined according to the embodiments of the present disclosure described above with reference to FIGS. 1 to 8. Furthermore, the display optimization information for the user may be determined based on at least one of user device attribute information, external environment information, and preference information.

For example, it is assumed that first, second and third users having different pieces of age information and the same eye examination information are present. For example, it is assumed that the age information of first user is twenties, the age information of the second user is thirties, and the age information of the third user is sixties. According to the embodiments of the present disclosure, the display settings of user devices related to the first user, the second user and the third user may be adjusted at least partially based on "different pieces of display optimization information" generated based on "different pieces of age information (i.e., different pieces of user information)."

In other words, FIG. 10(a) may show a user interface which is displayed on the user device of the first user, FIG. 10(b) may show a user interface which is displayed on the user device of the second user, and FIG. 10 (c) may show a user interface which is displayed on the user device of the third user.

According to the embodiments of the present disclosure, display optimization information for the user may be determined partially based on the eye examination information of the user, and an appropriate display environment may be provided to the user. For example, FIGS. 10(a), 10(b) and 10(c) may show pieces of content in which the sizes of fonts have been corrected based on presbyopia indices generated based on the pieces of presbyopia information of the users.

As another example, in a case where the same user uses the user device 100 in a room where a light is turned off during the day and in a case where the same user uses the user device 100 in a room where a light is turned on during the night, different pieces of display optimization information may be also determined.

Referring to FIGS. 10(a) to 10(c), for example, FIG. 10 (a) may show a user interface which is displayed on the display unit of the user device 100 in a room where a light is turned on during the day. FIG. 10(b) may show a user interface which is displayed on the display unit of the user device 100 in a room where a light is turned off during the day. Furthermore, FIG. 10(c) may show a user interface which is displayed on the display unit of the user device 100 in a room where a light is turned off during the night.

As another example, the brightness of a user interface displayed on a display unit may be controlled based on the light sensitivity of the user determined from preference information received from the user or eye examination information.

The above-described examples are merely examples according to the embodiments of the present disclosure, but the range of rights of the present disclosure is not limited thereto.

FIG. 11 are views showing display optimized for a user according to the embodiments of the present disclosure.

FIG. 11(a) shows an example of original content.

FIG. 11(b) shows the form in which the original content is viewed to the user according to the eye examination state of the user.

FIG. 11(c) shows the form in which content corrected according to the embodiments of the present disclosure is viewed to the user.

FIGS. 11(a) to 11(c) show examples of the content displayed on the user device according to the embodiments of the present disclosure. According to the embodiments of the present disclosure, the user may be provided with optimized content displayed on the user device without requiring a separate correction means.

From the comparison between FIG. 11(b) and FIG. 11(c), it can be seen that the content provided to the same user has been improved. Preferably, according to the embodiments of the present disclosure, an improvement may be made such that FIG. 11 (a) is the same as FIG. 11(c). For example, according to the embodiments of the present disclosure, FIG. 11 (b) may be corrected to FIG. 11 (c) in connection with at least one of the quantification of the difference between the original image and the image viewed to the user, one or more factors related to the eye examination information, and overall image quality recognized by the user.

Furthermore, FIG. 11 (b) may be corrected to correspond to FIG. 11 (a) based on at least one of a gradient descent-based conditional optimization algorithm, a van Cittert Zernike-based conditional optimization algorithm, and a wiener deconvolution algorithm.

Furthermore, the term "content" used herein includes at least one of an image, text, and a shape. The content displayed on FIGS. 11(a) to 11(c) includes all of an image, text, and a shape. At least one of an image, text, and a shape may be corrected based on preference received from the user. For example, when only the correction of text and a shape is preferred, the image may be minimally corrected (or may not be corrected), and the text and the shape may be corrected based on user eye examination information. The above-described description corresponds merely to examples according to the embodiments of the present disclosure, and it will be apparent to those skilled in the art that the present disclosure is not limited thereto.

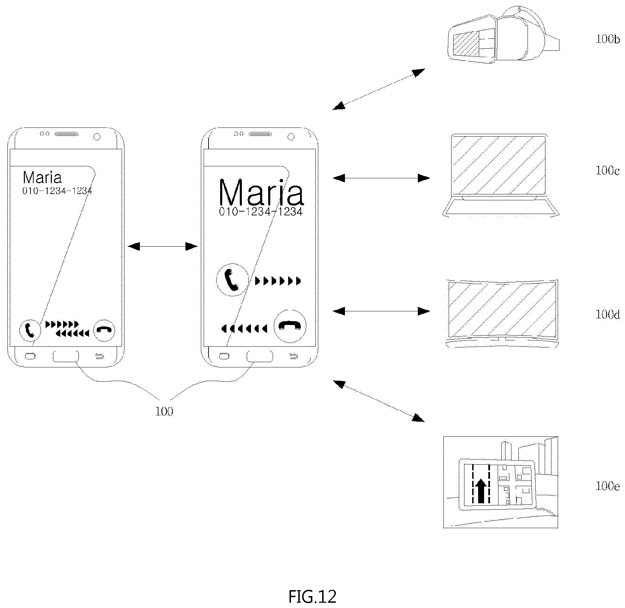

According to the above-described present disclosure, display can be optimized for the user. Furthermore, various types of information for display optimization may be provided to the user device and a server, thereby enabling the display settings of the at least one user-related user device to be also adjusted. This is described with reference to FIG. 12 below.

FIG. 12 is a view showing the way that one or more user devices are optimized based on the eye examination information of a user according to embodiments of the present disclosure.

According to the embodiments of the present disclosure, the display settings of the user device 100 may be adjusted at least partially based on display optimization information which is determined at least partially based on user information including the vision information of the user.

In greater detail, display optimization information may be determined at least partially based on the vision information of the user determined according to the embodiments of the present disclosure, and the display settings of the at least one user-related user devices 100b, 100c, 100d and 100e may be also adjusted at least partially based on the display optimization information.

According to the embodiments of the present disclosure, content display setting values determined based on logic predetermined for the correction of content to be displayed may be provided to at least one of the at least one user-related user device and the server, thereby allowing content to be displayed on the other user device to be corrected.

In other words, as shown in FIG. 12, once the vision of the user has been determined via the user device 100, thereby allowing content to be displayed on the at least one user-related user devices 100b, 100c, 100d and 100e to be corrected.

With the recent popularization of IT devices, the number of users each possessing a plurality of user devices has increased. According to the embodiments of the present disclosure, each of the plurality of user devices of each of the users may be automatically optimized without requiring that the user manually optimize each of the plurality of user devices.

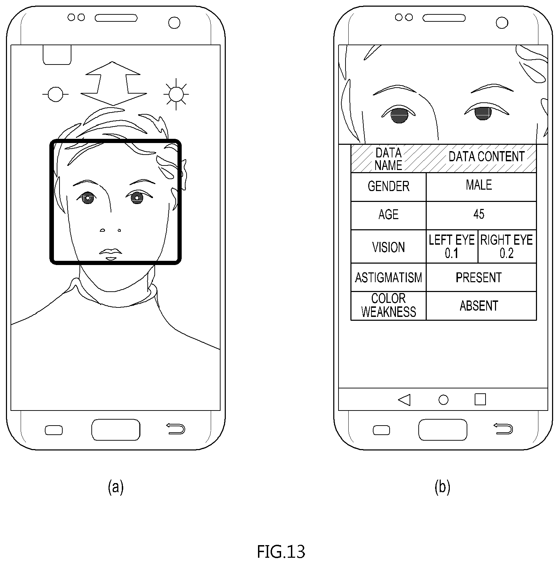

Referring to FIGS. 13 to 16, a user interface which is displayed on the user device 100 according to the embodiments of the present disclosure is now described. In this case, the user device 100 is described as a smartphone including at least one display unit, but the range of rights of the present disclosure is not limited thereto.

In the embodiments of the present disclosure, the user interface shown in FIG. 13 may display eye examination information including an acquired image of a pupil of the user and the vision information of the user analyzed from the image of the pupil.

In the embodiments of the present disclosure, the user interface shown in FIG. 13 may display a user interface configured to acquire eye examination information according to the embodiments of the present disclosure. The gender and age shown in FIG. 13 may be directly received from the user, or may be received from other server.

Referring to FIG. 13(a), an image acquired by photographing the user is displayed.

Referring to FIG. 13(b), an image of an eyeball including a pupil of the user is displayed. In the embodiments of the present disclosure, the image of the eyeball including the pupil is acquired from the image photographed by the photographing unit, and the vision information of the user may be determined from the image of the eyeball. The location of the eyeball including the pupil of the user may be determined from the image acquired by photographing the user, and various technologies may be adopted to acquire the image of the eyeball.

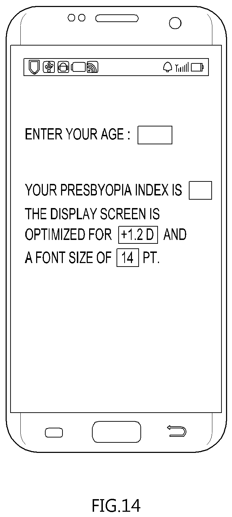

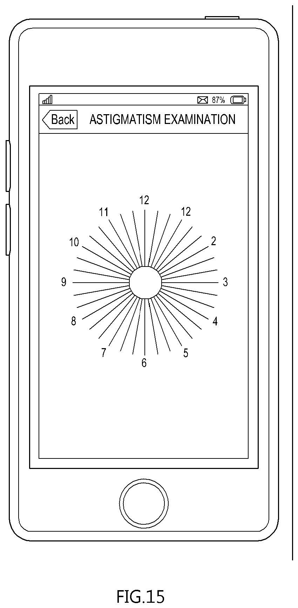

FIGS. 14 and 15 show other user interfaces which are displayed on a user device in order to acquire eye examination information according to embodiments of the present disclosure. The user interfaces shown in FIGS. 14 and 15 are merely examples of interfaces configured to check the vision and astigmatism of the user, but the range of rights of the present disclosure is not limited thereto.

The user interfaces shown in FIGS. 14 and 15 are presented to additionally acquire eye examination information other than the vision information determined according to the embodiments of the present disclosure.

According to the embodiments of the present disclosure, the eye examination information may be generated at least partially based on the responses of the user to an eye examination interface including at least one of an image and text adapted to perform the eye examination of the user. When the user interface is displayed on the display unit 180 in order to acquire the eye examination information of the user, the responses of the user may be implemented as, for example, voice inputs.

Furthermore, the user interface may be displayed on the display in order to receive the eye examination information from the user. For example, the user may input presbyopia age information via the user interface by referring to FIG. 14. Optionally or alternatively, the age input and presbyopia index in the user interface shown in FIG. 13 may be automatically input by referring to information previously received from the user (for example, at least one of previously input age information and previously generated eye examination information). Furthermore, part (for example, presbyopia age information) of the eye examination information displayed in the user interface shown in FIG. 14 may be determined based on the vision information determined according to the embodiments of the present disclosure.

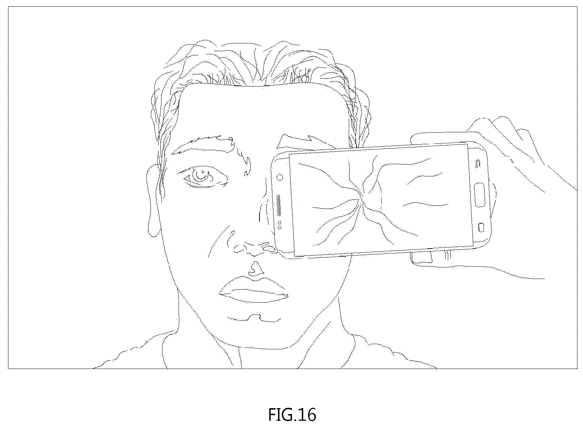

FIG. 16 shows a user interface which is displayed on a user device in order to authenticate a user according to the embodiments of the present disclosure.

Recently, as identity authentication methods, identify authentication methods using biometric identification information have been adopted in various technologies and/or devices. In this case, the identify authentication method using biometric identification information refer to an identity authentication method using biometric information regarding at least one of a signature, a face, an iris, a retina, a fingerprint, a voice, a hand shape, and hand blood vessels.

According to the embodiments of the present disclosure, the vision information of the user according to the embodiments of the present disclosure may be determined using an image of an eyeball acquired to perform identity authentication required for the use of the user device 100. In other words, according to the embodiments of the present disclosure, eye examination information including the vision information may be generated using the image of the eyeball acquired for identity authentication according to the embodiments of the present disclosure without intentionally acquiring the image of the eyeball, thereby improving the convenience of the user.

Furthermore, such images of the eyeball acquired for identity authentication required for the use of the user device 100 may be analyzed at predetermined period intervals. For example, analysis information including the eyeball health information of the user may be provided to the user based on the images of the eyeball at the predetermined period intervals.

Additionally, the shown and above-described interfaces are merely examples adapted to implement the embodiments of the present disclosure in the user device 100, but the range of rights of the present disclosure is not limited thereto. In other words, the components displayed on the interfaces shown in FIGS. 13 to 16 and described above may be omitted, added or changed according to the need of a person skilled in the art and/or the user.

The above descriptions are merely examples of at least some effects according to the embodiments of the present disclosure, and it will be apparent to those skilled in the art that the effects according to the range of rights of the present disclosure are not limited by the above descriptions.

Those skilled in the art of the present invention will appreciate that various exemplary logic blocks, modules, processors, means, circuits, and algorithm steps may be implemented using electronic hardware, various types of programs or design codes (designated as "software" herein for easy description), or a combination thereof described in association with the exemplary embodiments disclosed herein. In order to clearly describe the intercompatibility of the hardware and the software, various exemplary components, blocks, modules, circuits, and steps have been generally described above in association with functions thereof. Whether the functions are implemented as hardware or software depends on design restrictions given to a specific application and an entire system. Those skilled in the art of the present invention may implement functions described via various methods with respect to each specific application, but it should not be understood that the implementation determination departs from the scope of the present invention.

The logic blocks, the modules and the circuits described in connection with the disclosed embodiments may be implemented or performed using general-purpose processors, digital signal processors (DSPs), ASICs, field programmable gate arrays (FPGAs), programmable logic devices, discrete gates, transistor logics, discrete hardware components, or a combination thereof. The general-purpose processors may be microprocessors, but the processors may be typical processors, controllers, microcontrollers, or state machines. The processor may be implemented using a computing device, for example, a combination of a DSP and a microprocessor, a plurality of microprocessors, one or more microprocessors coupled to a DSP core, or other devices.

It will be appreciated that a specific order or a hierarchical structure of steps in the presented processes is one example of exemplary accesses. It will be appreciated that the specific order or the hierarchical structure of the steps in the processes within the scope of the present invention may be rearranged based on design priorities. The attached method claims provide elements of various steps in a sample order, but it does not mean that the method claims are limited to the presented specific order or hierarchical structure.

The steps of a method, process, or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of non-transitory computer-readable storage medium, media, or physical computer storage known in the art. An exemplary storage medium may be coupled to the processor such that the processor can read information from, and write information to, the storage medium. In the alternative, the storage medium may be integrated with the processor. The processor and the storage medium may reside in an ASIC. The ASIC may reside in a user terminal. In the alternative, the processor and the storage medium may reside as discrete components in a user terminal.

In one or more exemplary designs, the functions described may be implemented in hardware, software, firmware, or any combination thereof. If implemented in software, the functions may be stored on or transmitted over as one or more instructions or code on a computer-readable medium. Computer-readable media include both computer storage media and communication media including any medium that facilitates transfer of a computer program from one place to another. A storage media may be any available media that can be accessed by a computer. By way of example, and not limitation, such computer-readable media may include RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and blu-ray disc where disks usually reproduce data magnetically, while discs reproduce data optically with lasers. Combinations of the above should also be included within the scope of computer-readable media.

According to the embodiments of the present disclosure, the vision information of a user can be determined from an image of an eyeball of the user.

The description herein is presented to enable those skilled in the art to use and practice the invention. Various modifications to the disclosed embodiments will be readily apparent to those skilled in the art and the general principles defined herein may be applied to other embodiments and applications without departing from the spirit and scope of the present invention. Accordingly, the present invention is not intended to be limited to the disclosed embodiments, but should be interpreted in the broadest sense consistent with the principles and features disclosed herein.

## Claims

1. A computer program stored in a computer-readable storage medium including encoded instructions, wherein the computer program, when executed by one or more processors of a computer system, causes the one or more processors to perform operations, the operations comprising the operations of:

   allowing operation of a lighting unit to be controlled in response to a photographing request;
   allowing a photographing unit to acquire an image of a pupil of a user in response to the photographing request;
   allowing intensity profile information regarding a diameter of the pupil to be acquired from the image of the pupil;
   allowing crescent length information to be determined from the intensity profile information; and
   allowing vision information of the user to be determined at least partially based on the crescent length information.

2. The computer program of claim 1, wherein the operation of allowing a photographing unit to acquire an image of a pupil of a user comprises the operations of:

   allowing the image of the pupil of the user to be photographed in response to a program execution request; and
   allowing the image of the pupil of the user, photographed by the photographing unit in response to the photographing request, to be stored in response to the program execution request.

3. The computer program of claim 1, further comprising the operation of allowing a location of an eyeball including the pupil to be determined from the photographed image.

4. The computer program of claim 1, wherein the operation of allowing crescent length information to be determined comprises the operations of:

> analyzing a variation in intensity value intensity values from the intensity profile information; analyzing a direction of the variation; and determining at least one of vertical and horizontal lengths of a crescent at least partially based on the variation.

5. The computer program of claim 1, wherein the vision information of the user is determined additionally based on at least one of a radius length of the pupil, a distance from the photographing unit to the pupil, and a distance from the photographing unit to the lighting unit.

6. The computer program of claim 1, wherein the vision information of the user is determined additionally based on at least one of a PSF algorithm, an optical transfer function algorithm, an LSF algorithm, and a Strehl ratio algorithm.

7. The computer program of claim 1, further comprising the operation of determining optimization information of a display unit of a user-related user device at least partially based on the vision information of the user.

8. The computer program of claim 1, further comprising the operations of:

> allowing the photographing unit to acquire a second image of the pupil of the user in response to a second photographing request; comparing the image of the pupil acquired in response to the photographing request with the second image of the pupil acquired in response to the second photographing request; and determining whether the pupil has astigmatism at least partially based on the comparison; wherein the image of the pupil and the second image of the pupil are photographed and acquired in different directions.

9. A user device, comprising:

> at least one lighting unit; at least one photographing unit; a control unit configured to be allowed to control operation of the lighting unit in response to a photographing request; a photographed image acquisition unit configured to be allowed to allow the photographing unit to acquire an image of a pupil of a user in response to the photographing request; an intensity profile information acquisition unit configured to be allowed to acquire intensity profile information regarding a diameter of the pupil from the image of the pupil; a crescent length information determination unit configured to be allowed to determine crescent length information from the intensity profile information; and a vision information determination unit configured to be allowed to determine vision information of the user at least partially based on the crescent length information.

10. A server, comprising:

> an intensity profile information acquisition unit configured to be allowed to acquire intensity profile information regarding a diameter of a pupil from an image of the pupil of a user acquired in response to a photographing request; a crescent length information determination unit configured to be allowed to determine crescent length information from the intensity profile information; and a vision information determination unit configured to be allowed to determine vision information of the user at least partially based on the crescent length information.

11. The server of claim 10, wherein the image of the pupil of the user is acquired in response to operation of a lighting unit controlled in response to the photographing request.

**Amended claims in accordance with Rule 137(2) EPC.**

1. A computer program stored in a computer-readable storage medium including encoded instructions, wherein the computer program, when executed by one or more processors of a computer system, causes the one or more processors to perform operations, the operations comprising the operations of:

> controlling a lighting unit (110) in response to a photographing request; acquiring an image of a pupil of a user in response to the photographing request, through a photographing unit (120); acquiring intensity profile information regarding the pupil from the image of the pupil; determining crescent length information from the intensity profile information; and determining eye examination information of the user at least partially based on the crescent length information, **characterized by** further comprising:

> > determining optimization information of a display unit of a user-related user device at least partially based on the eye examination information of the user, and

adjusting display settings of the user device at least partially based on the determined display optimization information, and

wherein the optimization information includes at least one of font type information, font size information, font brightness information, front contrast information, font-to-screen ratio information, screen brightness information, image size information, image brightness information, resolution information, and color correction information.

2. The computer program of claim 1, wherein the operation of acquiring the image of the pupil of the user comprises the operations of:

photographing the image of the pupil of the user in response to a program execution request; and storing the image of the pupil of the user, photographed by the photographing unit (120) in response to the photographing request, in response to the program execution request.

3. The computer program of claim 1, further comprising the operation of determining a location of an eyeball including the pupil from the photographed image.

4. The computer program of claim 1, wherein the operation of determining crescent length information comprises the operations of:

analyzing a variation in intensity values from the intensity profile information;
analyzing a direction of the variation; and determining at least one of vertical and horizontal lengths of a crescent at least partially based on the variation.

5. The computer program of claim 1, wherein the eye examination information of the user is determined additionally based on at least one of a radius length of the pupil, a distance from the photographing unit (120) to the pupil, and a distance from the photographing unit (120) to the lighting unit (110).

6. The computer program of claim 1, wherein the eye examination information of the user is determined additionally based on at least one of a PSF algorithm, an optical transfer function algorithm, an LSF algorithm, and a Strehl ratio algorithm.

7. The computer program of claim 1, further comprising the operations of:

acquiring a second image of the pupil of the user in response to a second photographing request through the photographing unit (120);
comparing the image of the pupil acquired in response to the photographing request with the second image of the pupil acquired in response to the second photographing request; and determining whether the pupil has astigmatism at least partially based on the comparison;
wherein the image of the pupil and the second image of the pupil are photographed and acquired in different directions.

8. A user device, comprising:

at least one lighting unit (110);
at least one photographing unit (120);
a control unit (130) configured to control operation of the lighting unit (110) in response to a photographing request;
a photographed image acquisition unit (140) configured to acquire an image of a pupil of a user in response to the photographing request through the photographing unit (120);
an intensity profile information acquisition unit (150) configured to acquire intensity profile information regarding the pupil from the image of the pupil;
a crescent length information determination unit (160) configured to determine crescent length information from the intensity profile information; and
a eye examination information determination unit (170) configured to determine eye examination information of the user at least partially based on the crescent length information, **characterized in that** the user device is configured to determine optimization information of a display unit of a user-related user device at least partially based on the eye examination information of the user, and to adjust display settings of the user device at least partially based on the determined display optimization information, and
wherein the optimization information includes at least one of font type information, font size information, font brightness information, front contrast information, font-to-screen ratio information, screen brightness information, image size information, image brightness information, resolution information, and color correction information.

9. A server, comprising:

an intensity profile information acquisition unit (150) configured to acquire intensity profile information regarding a pupil from an image of the pupil of a user acquired in response to a photographing request;

a crescent length information determination unit (160) configured to determine crescent length information from the intensity profile information; and

a eye examination information determination unit (170) configured to determine eye examination information of the user at least partially based on the crescent length information, **characterized in that** the server is configured to determine optimization information of a display unit of a user-related user device at least partially based on the eye examination information of the user, and to adjust display settings of the user device at least partially based on the determined display optimization information, and

wherein the optimization information includes at least one of font type information, font size information, font brightness information, front contrast information, font-to-screen ratio information, screen brightness information, image size information, image brightness information, resolution information, and color correction information.

10. The server of claim 9, wherein the image of the pupil of the user is acquired in response to operation of a lighting unit (110) controlled in response to the photographing request.

FIG.1

100

| | |
|---|---|
| LIGHTING UNIT | ～110 |
| PHOTOGRAPHING UNIT | ～120 |
| CONTROL UNIT | ～130 |
| PHOTOGRAPHED IMAGE ACQUISITION UNIT | ～140 |
| INTENSITY PROFILE INFORMATION ACQUISITION UNIT | ～150 |
| CRESCENT LENGTH INFORMATION DETERMINATION UNIT | ～160 |
| VISION INFORMATION DETERMINATION UNIT | ～170 |
| MEMORY UNIT | ～180 |
| NETWORK UNIT | ～190 |

FIG.2

300

RECEPTION UNIT ~310

320

CONTROL UNIT

INTENSITY PROFILE INFORMATION ACQUISITION UNIT ~321

CRESCENT LENGTH INFORMATION DETERMINATION UNIT ~323

VISION INFORMATION DETERMINATION UNIT ~325

MEMORY UNIT ~330

NETWORK UNIT ~340

FIG.3

START

| ALLOW A LIGHT MODULE TO BE CONTROLLED | — S110 |

| ALLOW A PHOTOGRAPHED IMAGE INCLUDING AN IMAGE OF A PUPIL OF A USER TO BE ACQUIRED | — S120 |

| ALLOW INTENSITY PROFILE INFORMATION REGARDING THE DIAMETER OF THE PUPIL TO BE ACQUIRED FROM THE IMAGE OF THE PUPIL | — S130 |

| ALLOW CRESCENT LENGTH INFORMATION TO BE DETERMINED FROM THE INTENSIVE PROFILE INFORMATION | — S140 |

| ALLOW THE VISION INFORMATION OF THE USER TO BE DETERMINED | — S150 |

END

FIG.4

(a)          (b)          (c )

FIG.5

FIG.6

FIG.7

FIG.8

FIG.9

(a)  (b)  (c)

FIG.10

(a)                    (b)                    (c)

FIG.11

FIG.12

(a)

| DATA NAME | DATA CONTENT | |
|---|---|---|
| GENDER | MALE | |
| AGE | 45 | |
| VISION | LEFT EYE 0.1 | RIGHT EYE 0.2 |
| ASTIGMATISM | PRESENT | |
| COLOR WEAKNESS | ABSENT | |

(b)

FIG.13

ENTER YOUR AGE : [  ]

YOUR PRESBYOPIA INDEX IS [  ]
THE DISPLAY SCREEN IS
OPTIMIZED FOR [ +1.2 D ] AND
A FONT SIZE OF [ 14 ] PT.

FIG.14

FIG.15

FIG.16

Europäisches
Patentamt

European
Patent Office

Office européen
des brevets

# EUROPEAN SEARCH REPORT

Application Number

EP 17 18 1650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/036629 A2 (ICHECK HEALTH CONNECTION INC [US]; HUANG DAVID [US]; MURPHREE ALAN LIN) 14 March 2013 (2013-03-14) | 1,2,5, 8-11 | INV. G06T7/00 A61B3/00 G06K9/00 G06T7/64 |
| Y | * abstract * * page 3, line 28 - line 29 * * page 7, line 1 - line 5 * * page 8, line 9 - line 23 * * page 9, line 20 - line 21 * * page 13, line 30 - page 14, line 22 * * page 15, line 29 - line 31 * * page 16, line 18 - line 32 * * page 17, line 6 - line 15 * * page 29, line 15 - line 21 * ----- | 6,7 | |
| X | KWOK TIFFANY C K ET AL: "Democratizing Optometric Care: A Vision-Based, Data-Driven Approach to Automatic Refractive Error Measurement for Vision Screening", 2015 IEEE INTERNATIONAL SYMPOSIUM ON MULTIMEDIA (ISM), IEEE, 14 December 2015 (2015-12-14), pages 7-12, XP032886719, DOI: 10.1109/ISM.2015.55 [retrieved on 2016-03-25] | 1-5,9-11 | |
| Y | * Sec. III.B.1; paragraph [0001] - paragraph [0003] * * Sec. III.C.1; paragraph [0001] - paragraph [0003] * * Sec. III.A; paragraph [0003] * * figure 1 * ----- | 6,7 | |
|  | -/-- | | |

TECHNICAL FIELDS
SEARCHED (IPC)

G06T
A61B
G06K

The present search report has been drawn up for all claims

| Place of search | Date of completion of the search | Examiner |
|---|---|---|
| Munich | 1 September 2017 | Mohedano del Pozo, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| Europäisches Patentamt<br>European Patent Office<br>Office européen des brevets | **EUROPEAN SEARCH REPORT** | Application Number<br>EP 17 18 1650 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Larry N. Thibos ET AL: "Formation and Sampling of the Retinal Image"<br>In: "Seeing",<br>2000, Elsevier, XP055402209,<br>ISBN: 978-0-12-443760-9<br>pages 1-54, DOI:<br>10.1016/B978-012443760-9/50003-9,<br>* Sec. II.C and D * | 6 | |
| Y | US 2017/017083 A1 (SAMEC NICOLE ELIZABETH [US] ET AL) 19 January 2017 (2017-01-19)<br>* abstract *<br>* paragraph [0035] - paragraph [0038] *<br>* paragraph [1728] * | 7 | |

TECHNICAL FIELDS
SEARCHED       (IPC)

The present search report has been drawn up for all claims

| Place of search | Date of completion of the search | Examiner |
|---|---|---|
| Munich | 1 September 2017 | Mohedano del Pozo, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 18 1650

This annex lists the patent family members relating to the patent documents cited in the above-mentioned European search report.
The members are as contained in the European Patent Office EDP file on
The European Patent Office is in no way liable for these particulars which are merely given for the purpose of information.

01-09-2017

| Patent document cited in search report | | Publication date | Patent family member(s) | | Publication date |
|---|---|---|---|---|---|
| WO 2013036629 | A2 | 14-03-2013 | AU | 2012304575 A1 | 24-04-2014 |
| | | | CA | 2848206 A1 | 14-03-2013 |
| | | | EP | 2753228 A2 | 16-07-2014 |
| | | | JP | 6072798 B2 | 01-02-2017 |
| | | | JP | 2014526312 A | 06-10-2014 |
| | | | KR | 20140103900 A | 27-08-2014 |
| | | | US | 2013235346 A1 | 12-09-2013 |
| | | | WO | 2013036629 A2 | 14-03-2013 |
| US 2017017083 | A1 | 19-01-2017 | US | 2016270656 A1 | 22-09-2016 |
| | | | US | 2016287153 A1 | 06-10-2016 |
| | | | US | 2017000324 A1 | 05-01-2017 |
| | | | US | 2017000325 A1 | 05-01-2017 |
| | | | US | 2017000326 A1 | 05-01-2017 |
| | | | US | 2017000329 A1 | 05-01-2017 |
| | | | US | 2017000330 A1 | 05-01-2017 |
| | | | US | 2017000331 A1 | 05-01-2017 |
| | | | US | 2017000332 A1 | 05-01-2017 |
| | | | US | 2017000333 A1 | 05-01-2017 |
| | | | US | 2017000334 A1 | 05-01-2017 |
| | | | US | 2017000335 A1 | 05-01-2017 |
| | | | US | 2017000337 A1 | 05-01-2017 |
| | | | US | 2017000340 A1 | 05-01-2017 |
| | | | US | 2017000341 A1 | 05-01-2017 |
| | | | US | 2017000342 A1 | 05-01-2017 |
| | | | US | 2017000343 A1 | 05-01-2017 |
| | | | US | 2017000345 A1 | 05-01-2017 |
| | | | US | 2017000454 A1 | 05-01-2017 |
| | | | US | 2017000683 A1 | 05-01-2017 |
| | | | US | 2017001032 A1 | 05-01-2017 |
| | | | US | 2017007111 A1 | 12-01-2017 |
| | | | US | 2017007115 A1 | 12-01-2017 |
| | | | US | 2017007116 A1 | 12-01-2017 |
| | | | US | 2017007122 A1 | 12-01-2017 |
| | | | US | 2017007123 A1 | 12-01-2017 |
| | | | US | 2017007182 A1 | 12-01-2017 |
| | | | US | 2017007450 A1 | 12-01-2017 |
| | | | US | 2017007799 A1 | 12-01-2017 |
| | | | US | 2017007843 A1 | 12-01-2017 |
| | | | US | 2017010469 A1 | 12-01-2017 |
| | | | US | 2017010470 A1 | 12-01-2017 |
| | | | US | 2017017083 A1 | 19-01-2017 |
| | | | WO | 2016149416 A1 | 22-09-2016 |
| | | | WO | 2016149428 A1 | 22-09-2016 |

EPO FORM P0459

For more details about this annex : see Official Journal of the European Patent Office, No. 12/82